# EUROPEAN PATENT SPECIFICATION

(11) **EP 3 789 506 B1**
(45) Date of publication and mention of the grant of the patent: **18.10.2023**
(21) Application number: 18917319.8
(22) Date of filing: 19.12.2018
(51) Int. Cl.: C12Q 1/6895, C12N 15/11

(54) **PRUNUS MUME PENDULOUS TRAIT SNP MOLECULAR MARKERS AND USE THEREOF**
MOLEKULARE SNP-MARKER AUS HÄNGENDER PRUNUS MUME UND DEREN VERWENDUNG
MARQUEURS MOLÉCULAIRES SNP DU CARACTÈRE PLEUREUR DE PRUNUS MUME ET UTILISATION ASSOCIÉE

(30) Priority: 03.05.2018 CN 201810415993
(43) Date of publication of application: 10.03.2021
(73) Proprietor: Beijing Forestry University, Beijing 100083 (CN)
(72) Inventor: ZHANG, Qixiang, Beijing 100083 (CN); LI, Suzhen, Beijing 100083 (CN); ZHUO, Xiaokang, Beijing 100083 (CN); ZHENG, Tangchun, Beijing 100083 (CN); LI, Lulu, Beijing 100083 (CN); QIU, Like, Beijing 100083 (CN); SUN, Lidan, Beijing 100083 (CN); CHENG, Tangren, Beijing 100083 (CN); WANG, Jia, Beijing 100083 (CN)
(74) Representative: Hoffmann Eitle
(86) International application number: PCT/CN2018/121985
(87) International publication number: WO 2019/210696

(56) References cited:
- CN-A- 103 184 277
- CN-A- 108 715 902
- ZHANG JIE ET AL: "Genome-Wide Discovery of DNA Polymorphisms in Mei (Prunus mumeSieb. et Zucc.), an Ornamental Woody Plant, with Contrasting Tree Architecture and their Functional Relevance for Weeping Trait", PLANT MOLECULAR BIOLOGY REPORTER, SPRINGER SCIENCE+BUSINESS MEDIA B.V, NL, vol. 35, no. 1, 8 August 2016 (2016-08-08) , pages 37-46, XP036149765, ISSN: 0735-9640, DOI: 10.1007/S11105-016-1000-4 [retrieved on 2016-08-08]
- LIDAN SUN ET AL: "Genome-wide DNA polymorphisms in two cultivars of mei (Prunus mume sieb. et zucc.)", BMC GENETICS, BIOMED CENTRAL, GB, vol. 14, no. 1, 6 October 2013 (2013-10-06), page 98, XP021163119, ISSN: 1471-2156, DOI: 10.1186/1471-2156-14-98
- J. ZHANG ET AL: "High-density genetic map construction and identification of a locus controlling weeping trait in an ornamental woody plant (Prunus mume Sieb. et Zucc)", DNA RESEARCH, vol. 22, no. 3, 15 March 2015 (2015-03-15) , pages 183-191, XP055718417, JP ISSN: 1340-2838, DOI: 10.1093/dnares/dsv003
- ZHANG, . JIE: "Construction of . . High-Density Genetic Map and QTL Analysis of Ornametal Traits in Mei", China Doctoral Dissertations Full-Text Database (Agriculture Science and Technology), 15 August 2016 (2016-08-15), pages 1-146, XP009524795,

## Description

### Cross-reference to Related Application

This application claims the priority to the Chinese Patent Application No. 201810415993.7 entitled "SNP MOLECULAR MARKERS FOR WEEPING TRAIT OF MEI AND USE THEREOF" filed on May 3, 2018,

### Technical Field

The present invention relates to the fields of molecular biology and plant molecular selecting, and specifically, to a SNP molecular marker for weeping trait of Mei and use thereof.

### Background Art

Mei (*Prunus mume* Sieb.et Zucc.) belongs to family Rosaceae genus *Prunus* and is one of the ten famous traditional flowers in China. Mei has ornamental and use values, is originated from southwest China, and has a history of introduction and cultivation for more than 3,000 years (Chen Junyu. China Mei Flower Cultivars [M]. China Forestry Publishing House, 2010). There are various varieties of Mei with plenty of flower colors, flower types and plant types. Among them, weeping Mei (*Prunus mume* var. *pendula*) is one of the nine cultivars with unique plant type of Mei. Because of its unique tree posture and other ornamental traits such as flower color, floral scent and the like, weeping Mei is popular with people and has an important position in gardening.

Zhang Jie (Zhang Jie, Construction of High-Density Genetic Map and QTL Analysis for Some Ornamental Traits in Prunus mume [D]. Beijing Forestry University, 2016.) used SLAF-seq (Specific-Locus Amplified Fragment Sequencing) technology to develop genome-wide molecular markers for Mei, and constructed a genetic linkage map with the highest density of markers for Mei. The weeping trait was mapped to the 10.54 Mb to 11.68 Mb interval of chromosome 7, and ten SLAF molecular markers that could be closely linked to the weeping trait of Mei were obtained.

A large number of SNP markers are developed by high-throughput sequencing technology, and the developed trait-linked molecular markers are subjected to the initial screening of plants so as to achieve the purpose of molecular-assisted breeding, which can greatly shorten the breeding cycle and improve breeding efficiency. The present invention uses SLAF-seq technology to develop a large number of SNP markers for the weeping trait of Mei, screen out tightly linked molecular markers, and provide an important basis for its molecular marker-assisted selection breeding.

ZHANG JIE ET AL ("Genome-Wide Discovery of DNA Polymorphisms in Mei (Prunus mumeSieb. et Zucc.), an Ornamental Woody Plant, with Contrasting Tree Architecture and their Functional Relevance for Weeping Trait",PLANT MOLECULAR BIOLOGY REPORTER, vol. 35, no. 1, (2016-08-08), pages 37-46), describes SNP markers relating to weeping trait in Mei.

### Summary of the Invention

In order to solve the problems that exist in the prior art such as long screening cycle and low efficiency of the weeping trait of Mei, the object of the present invention is to provide a SNP molecular marker for weeping trait of Mei and use thereof.

In order to achieve the purpose of the present invention, the present invention uses the F₁ segregation population obtained by hybridization of "LIU BAN" Mei as the female parent and the "FENTAI CHUIZHI" Mei as the male parent, and cultivars of upright Mei and weeping Mei as test materials, to develop a Molecular marker for weeping trait of Mei and establish a marker-assisted selection system.

The SNP molecular marker for weeping trait of Mei provided by the present invention comprises molecular markers Marker301243 and/or Marker311414, and the nucleotide sequences of which are represented by SEQ ID NO. 1 and SEQ ID NO. 2, respectively.

The present invention also provides specific PCR primers for amplification of the molecular marker.

The primer sequences of the Marker301243 are as follows:
forward primer: 5'-TGAGAATGGACAATGAGCGT-3'
reverse primer: 5'-CTCTGCTGGACACCCCTAAT-3'.

The primer sequences of the Marker311414 are as follows:
forward primer: 5'-CTTAGGGAATGGTGTCGCTT-3'
reverse primer: 5'-AGAGCAGGCACCCAAGTAAGT-3'.

The present invention also provides the use of the molecular marker in identifying the weeping trait of Mei, comprising the following steps:
1) extracting genomic DNA of Mei to be tested;
2) performing a PCR amplification reaction using primers for amplification of the molecular marker with genomic DNA of the plant to be tested as a template;
3) the SNP locus corresponding to Marker301243 is located at the 426th base of the sequence represented by SEQ ID NO. 1, where the base is T or A, and the dominant allelotype of the weeping Mei is TT, while the dominant allelotype of the upright Mei is AT; the SNP locus corresponding to Marker311414 is located at the 607th base of the sequence represented by SEQ ID NO. 2, where the base is G or A, and the dominant allelotype of the weeping Mei is GG, while the dominant allelotype of the upright Mei is GA.

The volume of the PCR amplification system in step 2) is 20 µL, including: 2 µL of template DNA at a concentration of 50 ng/µL, 10 µL of 2 × Taq PCR Master Mix, 1 µL of forward primer at a concentration of 10 µmol/L and 1 µL of reverse primer at a concentration of 10 µmol/L, and 6 µL of ddH₂O.

In a specific embodiment of the present invention, the following PCR procedure is used as an example: pre-denaturing at 95°C for 2 min, 30 cycles of denaturing at 95°C for 20 s, annealing at 56°C for 30 s, extending at 72°C for 20 s; and final extending at 72°C for 5 min.

A person skilled in the art should know that the volume and/or amount of each component and the temperature and time of each reaction can be adjusted according to the different DNA polymerase used or other needs in the PCR amplification system and reaction procedure. Therefore, the choice of the PCR amplification system and reaction procedure according to the present invention includes, but is not limited to, the aforementioned amplification system and reaction procedure.

It should be pointed out that when using the two SNP molecular markers Marker301243 and Marker311414 provided by the present invention to identify the weeping trait of Mei or for molecular marker-assisted selection of Mei with weeping trait, a person skilled in the art may choose any one of Marker301243 and Marker311414 or a combination of two molecular markers Marker301243 and Marker311414 as requirements. The combination of the two markers can significantly improve the accuracy rate of identification.

Therefore, the primers of the molecular marker in step 2) may be forward and reverse primer pair of the Marker301243 and/or forward and reverse primer pair of the Marker311414.

The invention also provides AS-PCR primers for AS-PCR (allele-specific PCR) amplification of the molecular marker.

The AS-PCR primers are designed according to the SNP loci of Marker301243 (A/T) and Marker311414 (C/T), respectively. In order to improve the specificity of the primers, one mismatched base is also introduced at the 3^{rd} position from the 3' end of the specific primer, and the other primer (consensus primer) is designed according to a conventional method.

The primer sequences of the Marker301243 are as follows:
forward primer: 5'-TGGAAACTGAATAGATGCGAT-3'
reverse consensus primer: 5'-GGTGAAAGAGACATCAGAAAAT-3'
forward specific primer: 5'-TGGAAACTGAATAGATGCGAA-3'.

The primer sequences of the Marker311414 are as follows:
forward consensus primer: 5'-CATCTAAAATAAAATCTCAAAGG-3'
reverse primer: 5'-TCATAGGTATTCTTGTCTTTCTC-3'
reverse specific primer: 5'-TCATAGGTATTCTTGTCTTTCTT-3'.

The present invention also provides a method for screening or identifying the weeping trait of Mei, comprising the following steps:
1) extracting genomic DNA of Mei to be tested
2) performing an AS-PCR amplification using AS-PCR primers for amplification of the molecular marker with genomic DNA of the plant to be tested as a template;
3) detecting the product of the PCR amplification, and judging the corresponding SNP locus according to the presence or absence of the band of the product of the AS-PCR amplification, wherein corresponding to the SNP locus of the Marker301243, the dominant allelotype of the weeping Mei is TT, while the dominant allelotype of the upright Mei is AT; corresponding to the SNP locus of the Marker311414, the dominant allelotype of the weeping Mei is GG, while the dominant allelotype of the upright Mei is GA.

The volume of the AS-PCR amplification system in step 2) is 10 µL, including: 1 µL of template DNA at a concentration of 50 ng/µL, 5 µL of 2 × Taq PCR Master Mix, 0.5 µL of forward primer at a concentration of 10 µmol/L and 0.5 µL of reverse primer at a concentration of 10 µmol/L, and 3 µL of ddH₂O.

In a specific embodiment of the present invention, the following AS-PCR procedure is used as an example: pre-denaturing at 95°C for 2 min, 30 cycles of denaturing at 95°C for 20 s, annealing at 46-52°C for 30 s, extending at 72°C for 20 s; final extending at 72°C for 5 min.

A person skilled in the art should know that the volume and/or amount of each component and the temperature and time of each reaction can be adjusted according to the different DNA polymerase used or other needs in the PCR amplification system and reaction procedure. Therefore, the choice of the PCR amplification system and reaction procedure according to the present invention includes, but is not limited to, the aforementioned amplification system and reaction procedure.

The primers of the molecular marker in step 2) may be forward and reverse primer pair of the Marker301243 and/or forward and reverse primer pair of the Marker311414.

It is well known to a person skilled in the art that in the AS-PCR procedure, two AS-PCR primers need to be paired and used with a consensus primer respectively, and the same DNA template is subjected to PCR amplification in two PCR tubes. If the 3' end of the AS-PCR primer matches the allele in the sample, effective amplification can be performed; otherwise, effective amplification cannot be performed. In the present invention, when two specific primers of AS-PCR are paired with a consensus primer respectively for amplification, the genotype of the individual being amplified is identified, and according to the association between the genotype and the phenotype, the genotype frequency is counted to determine whether the SNP locus is closely related to the weeping trait or upright trait of Mei.

The present invention also provides a detection kit for screening or identifying the weeping trait of Mei comprising the specific PCR primers and/or the AS-PCR primers. The kit further comprises one or more of dNTPs, DNA polymerase, Mg²⁺, PCR reaction buffer, and standard positive template.

The present invention has the following advantages:
(1) The present invention uses SLAF-seq technology to develop a molecular marker linked to the weeping trait of Mei, and lays the foundation for molecular marker-assisted selection of Mei with weeping trait
(2) The SNP molecular marker obtained by the present invention shows good repeatability and stable amplification in the identification of populations and cultivars of Mei, and is not affected by environmental conditions.
(3) When the AS-PCR primers provided by the present invention are used in AS-PCR amplification reactions, the weeping trait and upright trait of Mei can be identified through electrophoresis detection, and the operation is simple.
(4) In the method for identifying the weeping trait of Mei provided by the present invention, through statistics of the genotype frequency, it was found that the two markers have high accuracy in the identification of F1 population and cultivars of Mei. Using Marker301243, the highest accuracy in the F1 population is 91.67%, and the accuracy in the upright individuals is up to 100%. At the same time, using the marker combination of Marker301243 + Marker311414, the accuracy in the F1 population is up to 100%. Using the marker combination of Marker301243 + Marker311414, the accuracy of identification in the cultivar of Mei is 89.13%, and the accuracy in the weeping cultivar of Mei is up to 100%.
(5) Using the two molecular markers screened out by the present invention for molecular marker-assisted selection can realize early selection at the seedling stage, reduce workload, greatly shorten the breeding cycle of Mei, and improve breeding efficiency.

### Brief Description of the Drawings

Figure 1 is sequencing peak patterns of the Marker301243 in Example 3 of the present invention, wherein panel A is the female parent 'LILT BAN' Mei, panel B is the male parent 'FENTAI CHUIZHI' Mei, panel C is upright Mei in the F1 generation and panel D is weeping Mei in the F1 generation.
Figure 2 is sequencing peak patterns of the Marker311414 in Example 3 of the present invention, wherein panel A is the female parent `LILT BAN' Mei, and panel B is the male parent `FENTAI CHUIZHI' Mei.
Figure 3 shows the results of AS-PCR amplification of Marker301243 (lanes 1 to 12 on the left of the electrophoresis marker (M)) and Marker311414 (lanes 1 to 12 on the right of the electrophoresis marker (M)) in the F1 population in Example 4 of the present invention, wherein lanes 1 to 6 are all weeping Mei, lanes 7 to 12 are all upright Mei, and M is Marker DL2000.
Figure 4 shows the results of AS-PCR amplification of Marker301243 in the female parent and the male parent and the weeping cultivar of Mei in Example 4 of the present invention, wherein lanes 1 to 2 are female parent `LILT BAN' Mei, lanes 3 to 4 are male parent 'FENTAI CHUIZHI' Mei, lanes 5 to 19 are weeping cultivar of Mei, and M is Marker DL2000.
Figure 5 shows the results of AS-PCR amplification of Marker301243 in the upright cultivar of Mei in Example 4 of the present invention, wherein lanes 1 to 27 are upright cultivar of Mei, and M is Marker DL2000.
Figure 6 shows the results of AS-PCR amplification of Marker311414 in the female parent and the male parent and the weeping cultivar of Mei in Example 4 of the present invention, wherein lanes 1 to 2 are female parent `LILT BAN' Mei, lanes 3 to 4 are male parent 'FENTAI CHUIZH' Mei, lanes 5 to 19 are weeping cultivar of Mei, and M is Marker DL2000.
Figure 7 shows the results of AS-PCR amplification of Marker311414 in the upright cultivar of Mei in Example 4 of the present invention, wherein lanes 1 to 27 are upright cultivar of Mei, and M is Marker DL2000.
Figure 8 is a comparison of the results of AS-PCR amplification of markers (Marker311414 (top) and Marker311414 (bottom)) respectively in the female parent and the male parent and the weeping cultivar of Mei in Example 4 of the present invention, wherein lanes 1 to 2 are female parent `LILT BAN' Mei, lanes 3 to 4 are male parent 'FENTAI CHUIZHI' Mei, lanes 5 to 19 are weeping cultivar of Mei, and M is Marker DL2000.
Figure 9 is a comparison of the results of AS-PCR amplification of markers (Marker301243 (top) and Marker311414 (bottom)) respectively in the upright cultivar of Mei in Example 4 of the present invention, wherein lanes 1 to 27 are upright cultivar of Mei, and M is Marker DL2000.
Figure 10 shows the accuracy of marker-assisted selection breeding of markers (Marker301243 and Marker311414) in the F1 population in Example 4 of the present invention.
Figure 11 shows the accuracy of marker-assisted selection breeding of markers (Marker301243 and Marker311414) in cultivars of Mei in Example 4 of the present invention.
Figure 12 shows the accuracy of marker-assisted selection breeding of the marker combination of Marker301243 + Marker311414 in the F1 population and cultivars of Mei in Example 4 of the present invention.

### Specific Modes for Carrying Out the Embodiments

Specific modes for carrying out the embodiments of the present invention will be further described in detail in combination with examples. It should be understood that the following Examples are given for illustrative purposes only, and are not intended to limit the scope of the present invention. A person skilled in the art can make various modifications and substitutions to the present invention without departing from the present invention.

Unless otherwise specified, the experimental methods used in the following Examples are conventional methods.

Unless otherwise specified, the materials, reagents, etc. used in the following Examples are all commercially available.

### Example 1: Development of a SNP molecular marker for weeping trait of Mei based on SLAF-seq technology

### 1. Experimental materials

The test materials included F1 segregation population obtained by hybridization of "LIU BAN" Mei as the female parent and "FENTAI CHUIZHI" Mei as the male parent, and the upright and weeping cultivars of Mei. The parents had significant differences in phenotypic characteristics such as weeping branches and flower color. All materials were planted in He Village, Moganshan Town, Huzhou City, Zhejiang Province (30.566389 °N, 119.879582 °E).

### 2. Extraction of genomic DNA

Twenty individual plants of the upright and weeping plant types in the F1 population were selected. DNA extraction was performed according to the instructions of the High-Efficiency Plant Genomic DNA Extraction Kit (Tiangen Biochemical Technology Co., Ltd.). The DNA integrity was measured using gel electrophoresis assay (1.0% agarose gel and 3 µL DNA mixed with 1 µL loading-buffer). The project was carried out for 15 min at a voltage of 150 V DNA concentration and purity were determined with NANO DROP 2000, ensuring that the DNA concentration was > 50 ng/µL.

### 3. Development of a molecular marker for the trait of Mei

Based on the genome information of Mei, the enzyme digestion prediction was performed to predict the number of molecular markers produced by different endonucleases on the Mei genome and their distribution on the genome. The two enzymes, *Hae* III and *Hpy* 166II (New England Biolabs, NEB, USA), were finally selected. Then the genomic DNA of Mei was digested with endonucleases *Hae* III and *Hpy* 166II. Then Klenow fragment (3'-5' exo) (NEB) and dATP were added. After incubation at 37°C, the digested DNA ends were connected to A bases, and a T4-ligase was used to link a double barcode tag linker (PAGE-purified, Life Technologies, USA) at the ends of the above-mentioned products with A bases. After the ligation was completed, the diluted DNA sample, dZTP, Q5^{®} high-fidelity enzyme and PCR primers were mixed for the subsequent PCR procedure. The PCR products were purified by a nucleic acid purification kit (Beckman Coulter, High Wycombe, UK) and then mixed. Then a 2% agarose gel was used to separate the mixed products of PCR fragments of different sizes. 6 µL of EB was added to 50 µL of the mixed DNA solution, and then the samples were loaded into the loading well. 6 µL of DL 1000 Maker was loaded at the same time. Electrophoresis was performed at 110 V for about 50 minutes. After electrophoresis, the gel was put into a BioRad UV imager (ChemiDoc^{™} MP). A band of 214 to 294 bp was cut off, and was put into a weighed centrifuge tube. The gel was purified and recovered with a QIAquick Gel Eztraction Kit. The recovered product was dissolved in 50 µL of EB solution. After the product recovered from the gel was diluted, HiSeq2500 sequencing platform of Illumina (Illumina, Inc; San Diego; CA, USA) was used to perform double-end 100 bp sequencing on the prepared SLAF library according to the instructions of the manufacturer.

### Example 2: Amplification of the SNP molecular marker

Primers were designed based on the differential markers for the weeping trait of Mei as follows:
According to Sanger sequencing results, two markers (Marker301243, Marker311414) were selected, and primers were designed using Primer Premier 5.0. The primer sequences were shown in Table 1.

**Table 1 Sequence information of two pair of primers for Sanger sequencing**

| The name of the primer | Primer sequence (5'-3') | | PCR product |
|---|---|---|---|
| | Forward primer | Reverse primer | |
| Slaf_01 | TGAGAATGGACAATGAG CGT | CTCTGCTGGACACCCCTAAT | 514 bp |
| Slaf_02 | CTTAGGGAATGGTGTCGC TT | AGAGCAGGCACCCAAGTAAG T | 653 bp |

Several upright and weeping individuals were randomly selected for PCR amplification. The volume of the PCR amplification system was 20 µL, including: 2 µL of template DNA at a concentration of 100 ng/µL, 10 µL of 2 × Taq PCR Master Mix (BIOMIGA), 1 µL of forward primer at a concentration of 10 µmol/L and 1 µL of reverse primer at a concentration of 10 µmol/L (SANGON Bioengineering Co., Ltd. Beijing Synthesis Department), and 10.3 µL of ddHzO. The PCR procedure included the following steps: pre-denaturing at 95°C for 2 min, 30 cycles of denaturing at 94°C for 20 s, annealing at 56°C for 30 s, extending at 72°C for 20 s; final extending at 72°C for 5 min. The PCR products were then subjected to Sanger sequencing (SANGON Beijing Biotech Engineering Co., Ltd. Beijing Sequencing Department). The sequencing results of progeny were compared with that of the parents to ensure that the marker included true SNP locus which was closely linked to the weeping trait.

In the above two Examples, based on quantitative trait locus (QTL) analysis and SLAP-index analysis, Marker301243 and/or Marker311414 were found to be significantly associated with the weeping trait of Mei. Through PCR amplification and Sanger sequencing, Marker301243 and/or Marker311414 were identified to have true SNP loci which were isolated in the progeny.

### Example 3: Establishment of an AS-PCR method for genotyping based on SNP markers

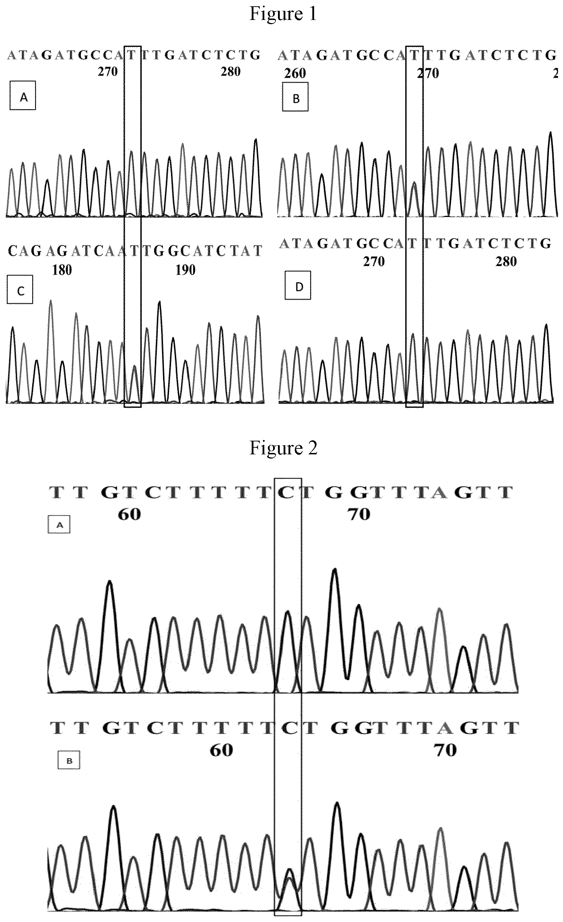

Two markers were obtained by Sanger sequencing (i.e. Marker301243 and Marker311414, the nucleotide sequences of which are represented by SEQ ID NO.1 and SEQ ID NO.2, respectively). The AS-PCR primers were designed for the corresponding SNP loci. The allele-specific design was based on the SNP loci of Marker301243 (A/T) and Marker311414 (C/T), respectively. In order to improve the specificity of the primers, one mismatched base was introduced at the 3^{rd} position from the 3' end of the specific primer, and the other primer (consensus primer) was designed according to a conventional method. The specificity of the designed primers was detected with NCBI (Table 2). In the AS-PCR procedure, two AS-PCR primers need to be paired and used with a consensus primer respectively, and the same DNA template was subjected to PCR amplification in two PCR tubes. If the 3' end of the AS-PCR primer matched the allele in the sample, effective amplification can be performed; otherwise, effective amplification cannot be performed. The volume of the AS-PCR amplification system was 10 µL, including: 1 µL of template DNA at a concentration of 50 ng/µL, 5 µL of 2 × Taq PCR Master Mix, 0.5 µL of forward primer at a concentration of 10 µmol/L and 0.5 µL of reverse primer at a concentration of 10 µmol/L, and 3 µL of ddH₂O. The PCR procedure included the following steps: pre-denaturing at 95°C for 2 min, 30 cycles of denaturing at 95°C for 20 s, annealing at 46-52°C for 30 s, extending at 72°C for 20 s; final extending at 72°C for 5 min. After the amplification was completed, PCR products were detected by 1% agarose gel electrophoresis, and the genotypes of individual plants of the F1 population were counted to determine the dominant allelotype of the weeping trait or upright trait of Mei. Among them, the SNP locus corresponding to Marker301243 is located at the 426th base of the sequence represented by SEQ ID NO. 1, where the base is T or A (Figure 1), and the dominant allelotype of the weeping trait of Mei is TT, while the dominant allelotype of the upright trait of Mei is AT; the SNP locus corresponding to Marker311414 is located at the 607th base of the sequence represented by SEQ ID NO. 2, where the base is G or A, and the dominant allelotype of the weeping trait of Mei is GG, while the dominant allelotype of the upright trait of Mei is GA (Figure 2).

The sequence information of the two pairs of primers of the markers used for AS-PCR amplification was shown in Table 2.

**Table 2 Sequence information of the two pairs of primers of the markers for AS-PCR amplification**

| The name of the primer | Primer sequence (5'-3') | | PCR product |
|---|---|---|---|
| | Forward primer | Reverse primer | |
| AS_01 | Ref: TGGAAACTGAATAGATG**C**CAT | | 574 bp |
| | F: TGGAAACTGAATAGATGC**G**A**T** | GGTGAAAGAGACATCAGAAAAT | |
| | S: TGGAAACTGAATAGATGC**G**A**A** | | |
| AS_02 | | Ref: TCATAGGTATTCTTGTCTTT**T**T**C** | 519 bp |
| | CATCTAAAATAAAATCTCAAAGG | R: TCATAGGTATTCTTGTCTTT**C**T**C** | |
| | | S: TCATAGGTATTCTTGTCTTT**C**T**T** | |

Note: Ref is the reference genome sequence; F is the forward primer sequence; R is the reverse primer sequence; S is the specific primer sequence; bases in bold font are differential bases in the AS-PCR specific primer.

### Example 4: Application of molecular markers (Marker301243 and Marker311414)

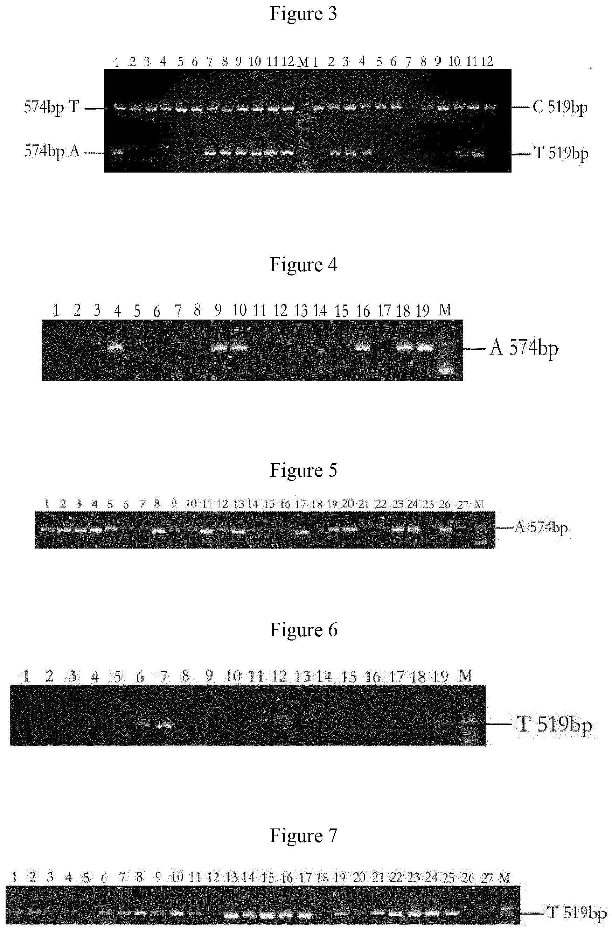

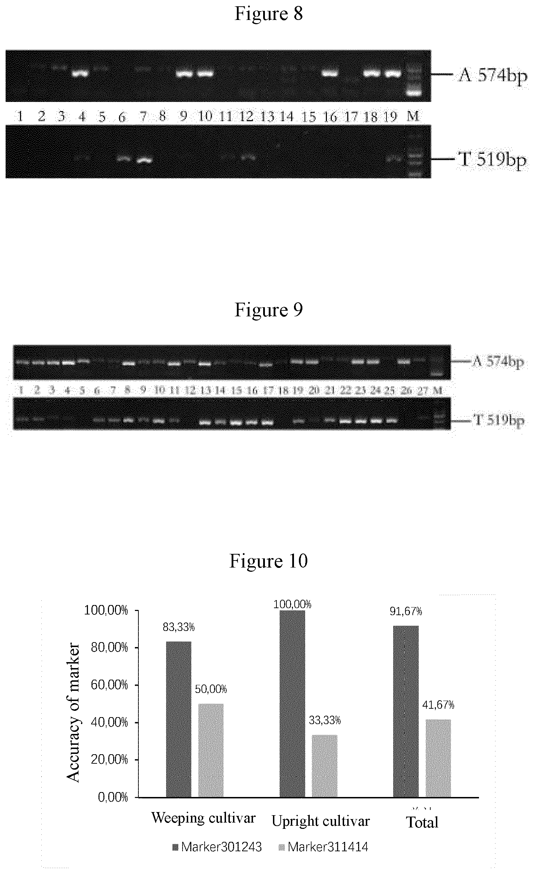

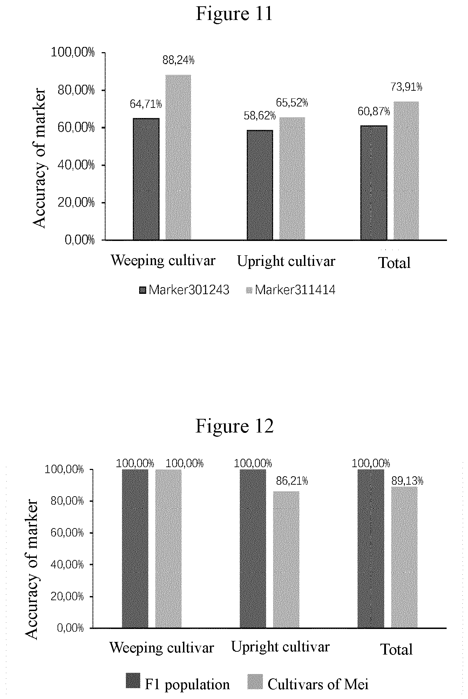

The accuracy, stability and reproducibility of molecular markers (Marker301243 and Marker311414) were measured and the results were as follows:
The two markers (Marker301243 and Marker311414) were used to perform AS-PCR amplification verification on the F1 population. The identification results of the markers were shown in Figures 3 and 10. The highest accuracy of Marker301243 in the F1 population was 91.67%, and the accuracy in the upright individuals was up to 100%. When the marker combination of Marker301243 + Marker311414 was used for phenotypic identification, the success rate in the F1 population was up to 100% (Figure 12). It can be seen that the developed SNP markers can be used in the molecular marker-assisted selection breeding of the weeping trait in F1 populations and cultivars of Mei.

The stability of the obtained specific molecular marker is very important for its application. The same primers were used in the amplification of the F1 population and cultivars of Mei, if a stable band can be obtained, it means that the developed marker is stable and can be used for identification of different plant types of Mei. The stabilities and accuracy of the developed SNP markers were verified with cultivars of Mei as materials. Taking Marker301243 as an example, two bands were obtained through amplification in the upright individuals which are of AT allelotype, and one band was obtained through amplification in weeping individuals, which is of TT allelotype, indicating that both markers can be better used to identify different phenotypes in cultivars of Mei and has good stability (Figure 3). Further, the specificity and stability of molecular markers (Marker301243 and Marker311414) were verified in the weeping and upright cultivars of Mei, respectively. For the allelotype of the two molecular markers, there may be three kinds of genotype (AA, TT, and AT for Marker301243; AA (TT), GG (CC), and AG (CT) for Marker311414). However, when a pair of primers (a specific primer and a consensus primer) was used for amplification (Figure 4 to 9), a band can be obtained through amplification only in the individuals with the AA or AT genotype paired with the specific primer used, and no band was obtained through amplification in the individuals with the TT genotype (taking Marker301243 as an example). Figure 4 shows the test results of Marker301243 in the weeping cultivar, wherein, those without bands are the weeping cultivar with TT genotypes. Figure 6 shows the test results of Marker311414 in the weeping cultivar, wherein, those without bands are weeping cultivar with GG (CC) genotypes. Figure 8 shows the test results of the combination of Marker301243/Marker311414, and the corresponding genotypes can be expressed as TT/CC (with band/without band), A_/CC (with band/without band), and TT/_T (with band/without band). It can be seen from Figure 8 that most of the weeping individuals were homozygous, showing a TT/CC genotype, and there was no band in amplification. In the same way, the dominant genotype of SNP locus in the upright cultivar was AT, and a band can be obtained through amplification using specific primers. Figure 5 shows the test results of Marker 30124 in the upright cultivar. It can be seen from the figure that most of the upright cultivar showed bands in amplification and were of AT genotype. Figure 7 shows the test results of Marker311414 in the upright cultivar. Most upright cultivar showed bands in the amplification and were of CT genotype. Figure 9 shows the test results of the combination of Marker301243/Marker311414. The corresponding genotypes can be expressed as A _/_ T (with band/with band), A_/CC (with band/without band), TT/_T (without band/with band), and TT/CC (without band/without band). Therefore, through the statistics of the specific band types of Marker301243/Marker311414, the corresponding identification results can be obtained. It can be seen from Figure 9 that most of the upright individuals are heterozygous, showing a combination of AT/CT genotypes, and there were two bands in amplification. A few upright individuals show the same genotype as that of the weeping trait, and there was one band or no band in the amplification (as shown in Figure 9, Individual 18). It can be seen that the molecular markers (Marker301243 and Marker311414) have high stability and reliability in the AS-PCR amplification in the cultivars of Mei (Figures 4 to 9). Marker301243 has a significantly higher accuracy in the F1 population than that in the cultivars of Mei, while Marker311414 does the opposite (Figure 11). When the marker combination Marker301243 + Marker311414 was used for phenotypic identification, the success rate was 89.13% in the cultivars of Mei, and 100% in the weeping cultivar of Mei (Figure 12).

Figure 3 shows the results of AS-PCR amplification of markers (Marker301243 and Marker311414) in the F1 population. Figures 4 to 9 show the stability of AS-PCR amplification of Marker301243 and Marker311414 in the cultivars of Mei.

The molecular marker-assisted selection breeding established by the present disclosure can realize early selection at the seedling stage, reduce workload, improve the selection efficiency of the weeping trait of Mei, and greatly shorten the breeding cycle.

Although the present invention has been described in detail with the general description and specific embodiments, it is obvious to a person skilled in the art that some modifications or improvements can be made based on the present invention. Therefore, these modifications or improvements made without departing from the present invention belong to the scope of protection of the present invention.

### Industrial applicability

The present invention provides a SNP molecular marker for weeping trait of Mei and use thereof. The SNP molecular marker for weeping trait of Mei provided by the present invention specifically include molecular markers (Marker301243 and/or Marker311414), the nucleotide sequences of which are represented by SEQ ID NO. 1 and SEQ ID NO. 2, respectively. The present invention uses SLAF-seq technology to develop and obtain two SNP molecular marker for weeping trait of Mei (Marker301243 and Marker311414), and provides primers for sequence amplification and primers for allele-specific PCR. The molecular markers (Marker301243 and/or Marker311414) and their primers have high accuracy, stability and good repeatability when used for the identification of cultivars of Mei. Using the two molecular markers for molecular marker-assisted breeding, can realize early selection at the seedling stage, quickly identify of the weeping trait of Mei, reduce workload, greatly shorten the breeding time of Mei, and have good economic value and application prospects.

## Claims

1. A SNP molecular marker for weeping trait of Mei, comprising molecular markers: Marker301243 and/or Marker311414, the nucleotide sequences of which are represented by SEQ ID NO. 1 and SEQ ID NO. 2, respectively.

2. Specific PCR primers for amplification of the molecular marker according to claim 1, wherein,
the primer sequences of the Marker301243 are as follows:
forward primer: 5'-TGAGAATGGACAATGAGCGT-3'
reverse primer: 5'-CTCTGCTGGACACCCCTAAT-3';
the primer sequences of the marker Marker311414 are as follows:
forward primer: 5'-CTTAGGGAATGGTGTCGCTT-3'
reverse primer: 5'-AGAGCAGGCACCCAAGTAAGT-3'.

3. Use of the molecular marker according to claim 1 in identifying the weeping trait of Mei, comprising the following steps:
1) extracting genomic DNA of Mei plant to be tested;
2) performing a PCR amplification reaction using primers of the molecular marker according to claim 1 with genomic DNA of the plant to be tested as a template;
3) the SNP locus corresponding to Marker301243 is located at the 426th base of the sequence represented by SEQ ID NO. 1, where the base is T or A, and the dominant allelotype of the weeping Mei is TT, while the dominant allelotype of the upright Mei is AT; the SNP locus corresponding to Marker311414 is located at the 607th base of the sequence represented by SEQ ID NO. 2, where the base is G or A, and the dominant allelotype of the weeping Mei is GG, while the dominant allelotype of the upright Mei is GA.

4. The use according to claim 3, wherein, the volume of the PCR amplification system in step 2) is 20 µL, comprising: 2 µL of template DNA at a concentration of 50 ng/µL, 10 µL of 2 × PCR Master Mix, 1 µL of forward primer at a concentration of 10 µmol/L and 1 µL of reverse primer at a concentration of 10 µmol/L, and 6 µL of ddH₂O.

5. AS-PCR primers for AS-PCR amplification of the molecular marker according to claim 1, wherein,
the primer sequences of the Marker301243 are as follows:
forward primer: 5'-TGGAAACTGAATAGATGCGAT-3',
reverse consensus primer: 5'-GGTGAAAGAGACATCAGAAAAT-3',
forward specific primer: 5'-TGGAAACTGAATAGATGCGAA-3';
the primer sequences of the Marker311414 are as follows:
forward consensus primer: 5'-CATCTAAAATAAAATCTCAAAGG-3',
reverse primer: 5'-TCATAGGTATTCTTGTCTTTCTC-3',
reverse specific primer: 5'-TCATAGGTATTCTTGTCTTTCTT-3'.

6. A method for screening or identifying the weeping trait of Mei, comprising the following steps:
1) extracting genomic DNA of Mei plant to be tested;
2) performing an AS-PCR amplification using AS-PCR primers of the molecular marker according to claim 1 with genomic DNA of the plant to be tested as a template;
3) detecting the product of the PCR amplification, and judging the corresponding SNP locus according to the presence or absence of the band of the product of the AS-PCR amplification, wherein corresponding to the SNP locus of the Marker301243, the dominant allelotype of the weeping Mei is TT, while the dominant allelotype of the upright Mei is AT; corresponding to the SNP locus of the Marker311414, the dominant allelotype of the weeping Mei is GG, while the dominant allelotype of the upright Mei is GA.

7. The method according to claim 6, wherein, the volume of the AS-PCR amplification system in step 2) is 10 µL, comprising: 1 µL of template DNA at a concentration of 50 ng/µL, 5 µL of 2 × Taq PCR Master Mix, 0.5 µL of forward primer at a concentration of 10 µmol/L and 0.5 µL of reverse primer at a concentration of 10 µmol/L, and 3 µL of ddH₂O.

8. A detection kit for screening or identifying the weeping trait of Mei comprising the PCR primers according to claim 2 and/or claim 5.

9. The kit according to claim 8, wherein, the kit further comprises one or more of dNTPs, DNA polymerase, Mg²⁺, PCR reaction buffer, and standard positive template.

## Patentansprüche

1. Molekularer SNP-Marker für *Prunus mume* Var. *Pendula* (weeping trait of Mei), umfassend die molekularen Marker:
Marker301243 und/oder Marker311414, deren Nukleotidsequenzen durch SEQ ID NO. 1 bzw. SEQ ID NO. 2 dargestellt werden.

2. Spezifische PCR-Primer für eine Amplifikation des molekularen Markers gemäß Anspruch 1, wobei,
die Primer-Sequenzen des Marker301243 wie folgt sind:
Vorwärtsprimer: 5'- TGAGAATGGACAATGAGCGT-3'
Rückwärtsprimer: 5'- CTCTGCTGGACACCCCTAAT-3';
die Primer-Sequenzen des Markers Marker311414 wie folgt sind:
Vorwärtsprimer: 5'-CTTAGGGAATGGTGTCGCTT-3'
Rückwärtsprimer: 5'-AGAGCAGGCACCCAAGTAAGT-3'.

3. Verwendung des molekularen Markers gemäß Anspruch 1 zur Identifizierung der *Prunus mume* Var. *Pendula* (weeping trait of Mei), umfassend die folgenden Schritte:
1) Extrahieren der genomischen DNA einer zu testenden *Prunus mume* (Mei) Pflanze;
2) Durchführen einer PCR-Amplifikationsreaktion unter Verwendung von Primern des molekularen Markers gemäß Anspruch 1 mit genomischer DNA der zu testenden Pflanze als ein Templat;
3) der SNP-Locus, der dem Marker301243 entspricht, befindet sich an der 426. Base der durch SEQ ID NO. 1 dargestellten Sequenz, wobei die Base T oder A ist, und der dominante Allel-Typ der *Prunus mume* Var. *Pendula* (weeping Mei) TT ist, während der dominante Allel-Typ der *Prunus mume* Var. *Typica* (upright Mei) AT ist; der SNP-Locus, der dem Marker311414 entspricht, befindet sich an der 607. Base der durch SEQ ID NO. 2 dargestellten Sequenz, wobei die Base G oder A ist, und der dominante Allel-Typ der *Prunus mume* Var. *Pendula* (weeping Mei) GG ist, während der dominante Allel-Typ der *Prunus mume* Var. *Typica* (upright Mei) GA ist.

4. Verwendung gemäß Anspruch 3, wobei, das Volumen des PCR-Amplifikationssystems in Schritt 2) 20 µL ist, umfassend: 2 µL Templat-DNA in einer Konzentration von 50 ng/µL, 10 µL 2 × PCR Master Mix, 1 µL Vorwärtsprimer in einer Konzentration von 10 µmol/L und 1 µL Rückwärtsprimer in einer Konzentration von 10 µmol/L, und 6 µL ddHzO.

5. AS-PCR-Primer für die AS-PCR-Amplifikation des molekularen Markers gemäß Anspruch 1, wobei,
die Primer-Sequenzen des Marker301243 wie folgt sind:
Vorwärtsprimer: 5'-TGGAAACTGAATAGATGCGAT-3',
Konsensus-Rückwärtsprimer: 5'-GGTGTGAAAGAGACATCAGAAAAT-3',
spezifischer Vorwärtsprimer: 5'-TGGGAAACTGAATAGATGCGAA-3';
die Primer-Sequenzen des Marker311414 wie folgt sind:
Konsensus-Vorwärtsprimer: 5'-CATCTAAAATAAAATCTCAAAGG-3', Rückwärtsprimer: 5'-TCATAGGTATTCTTGTCTTTCTC-3',
spezifischer Rückwärtsprimer: 5'-TCATAGGTATTCTTGTCTTTCTT-3'.

6. Verfahren zum Screening oder zur Identifizierung der *Prunus mume* Var. *Pendula* (weeping trait of Mei), umfassend die folgenden Schritte:
1) Extrahieren der genomischen DNA einer zu testenden *Prunus mume* (Mei) Pflanze;
2) Durchführen einer AS-PCR-Amplifikation unter Verwendung von AS-PCR-Primern des molekularen Markers gemäß Anspruch 1 mit genomischer DNA der zu testenden Pflanze als ein Templat;
3) Detektieren des Produkts der PCR-Amplifikation, und Beurteilen des entsprechenden SNP-Locus gemäß dem Vorhandensein oder Abwesendsein der Bande des Produkts der AS-PCR-Amplifikation, wobei entsprechend dem SNP-Locus des Marker301243, der dominante Allel-Typ der *Prunus mume* Var. *Pendula* (weeping Mei) TT ist, während der dominante Allel-Typ der *Prunus mume* Var. *Typica* (upright Mei) AT ist; entsprechend dem SNP-Locus des Marker311414, der dominante Allel-Typ der *Prunus mume* Var. *Pendula* (weeping Mei) GG ist, während der dominante Allel-Typ des *Prunus mume* Var. *Typica* (upright Mei) GA ist.

7. Verfahren gemäß Anspruch 6, wobei, das Volumen des AS-PCR-Amplifikationssystems in Schritt 2) 10 µL ist, umfassend: 1 µL Templat-DNA in einer Konzentration von 50 ng/µL, 5 µL 2 × Taq PCR Master Mix, 0,5 µL Vorwärtsprimer in einer Konzentration von 10 µmol/L und 0,5 µL Rückwärtsprimer in einer Konzentration von 10 µmol/L, und 3 µL ddHzO.

8. Detektions-Kit zum Screening oder zur Identifizierung der *Prunus mume* Var. *Pendula* (weeping trait of Mei) umfassend die PCR-Primer gemäß Anspruch 2 und/oder Anspruch 5.

9. Kit gemäß Anspruch 8, wobei, das Kit ferner eines oder mehrere umfasst von dNTPs, DNA-Polymerase, Mg²⁺, PCR-Reaktionspuffer, und Standard-Positiv-Templat.

## Revendications

1. Marqueur moléculaire SNP du caractère pleureur du Mei, comprenant les marqueurs moléculaires : Marqueur 301243 et/ou Marqueur 311414, les séquences nucléotidiques de ceux-ci étant respectivement représentées par SEQ ID NO: 1 et SEQ ID NO : 2.

2. Amorces de PCR spécifiques pour l'amplification du marqueur moléculaire selon la revendication 1, dans lesquelles,
les séquences d'amorces du Marqueur 301243 sont les suivantes :
amorce sens : 5'-TGAGAATGGACAATGAGCGT-3'
amorce anti-sens : 5'-CTCTGCTGGACACCCCTAAT-3' ;
les séquences d'amorces du Marqueur 311414 sont les suivantes :
amorce sens : 5'-CTTAGGGAATGGTGTCGCTT-3'
amorce anti-sens : 5'-AGAGCAGGCACCCAAGTAAGT-3'.

3. Utilisation du marqueur moléculaire selon la revendication 1 pour identifier le caractère pleureur du Mei, comprenant les étapes suivantes :
1) extraction de l'ADN génomique de la plante Mei à tester ;
2) réalisation d'une réaction d'amplification PCR en utilisant les amorces du marqueur moléculaire selon la revendication 1 avec l'ADN génomique de la plante à tester comme modèle ;
3) le locus SNP correspondant au Marqueur 301243 est situé à la 426e base de la séquence représentée par SEQ ID NO. 1, où la base est T ou A, et l'allélotype dominant du Mei pleureur est TT, tandis que l'allélotype dominant du Mei dressé est AT ; le locus SNP correspondant au Marqueur 311414 est situé à la 607e base de la séquence représentée par SEQ ID NO. 2, où la base est G ou A, et l'allélotype dominant du Mei pleureur est GG, tandis que l'allélotype dominant du Mei dressé est GA.

4. Utilisation selon la revendication 3, dans laquelle, le volume du système d'amplification PCR à l'étape 2) est de 20 µL, comprenant : 2 µL d'ADN matrice à une concentration de 50 ng/µL, 10 µL de 2 × PCR Master Mix, 1 µL d'amorce sens à une concentration de 10 µmol/L et 1 µL d'amorce anti-sens à une concentration de 10 µmol/L, et 6 µL de ddH₂0.

5. Amorces AS-PCR pour l'amplification AS-PCR du marqueur moléculaire selon la revendication 1, dans lesquelles,
les séquences d'amorces du Marqueur 301243 sont les suivantes :
amorce sens : 5'-TGGAAACTGAATAGATGCGAT-3',
amorce de consensus anti-sens : 5'-GGTGAAAGAGACATCAGAAAAT-3',
amorce spécifique sens : 5'-TGGAAACTGAATAGATGCGAA-3' ;
les séquences d'amorces du Marqueur 311414 sont les suivantes :
amorce de consensus sens : 5'-CATCTAAAATAAAATCTCAAAGG-3',
amorce anti-sens : 5'-TCATAGGTATTCTTGTCTTTCTC-3',
amorce spécifique anti-sens : 5'-TCATAGGTATTCTTGTCTTTCTT-3'.

6. Procédé de dépistage ou d'identification du caractère pleureur du Mei, comprenant les étapes suivantes :
1) extraction de l'ADN génomique de la plante Mei à tester ;
2) réalisation d'une réaction d'amplification AS-PCR en utilisant les amorces AS-PCR du marqueur moléculaire selon la revendication 1 avec l'ADN génomique de la plante à tester comme modèle ;
3) détection du produit de l'amplification PCR et évaluation du locus SNP correspondant en fonction de la présence ou de l'absence de la bande du produit de l'amplification AS-PCR, dans lequel, en ce qui concerne le locus SNP du marqueur 301243, l'allélotype dominant du Mei pleurer est TT, tandis que l'allélotype dominant du Mei dressé est AT ; en ce qui concerne le locus SNP du marqueur 311414, l'allélotype dominant du Mei pleureur est GG, tandis que l'allélotype dominant du Mei dressé est GA.

7. Procédé selon la revendication 6, dans lequel, le volume du système d'amplification AS-PCR à l'étape 2) est de 10 µL, comprenant : 1 µL d'ADN matrice à une concentration de 50 ng/µL, 5 µL de 2 × Taq PCR Master Mix, 0,5 µL d'amorce sens à une concentration de 10 µmol/L et 0,5 µL d'amorce anti-sens à une concentration de 10 µmol/L, et 3 µL de ddH₂0.

8. Kit de détection pour le dépistage ou l'identification du caractère pleureur du Mei comprenant les amorces PCR selon la revendication 2 et/ou la revendication 5.

9. Kit selon la revendication 8, dans lequel le kit comprend en outre un ou plusieurs dNTP, de l'ADN polymérase, du Mg²⁺, du tampon de réaction PCR et un modèle positif standard.
